# EUROPÄISCHE PATENTSCHRIFT

(11) **EP 1 685 255 B1**
(45) Veröffentlichungstag und Bekanntmachung des Hinweises auf die Patenterteilung: **02.05.2012**
(21) Anmeldenummer: 04803117.3
(22) Anmeldetag: 10.11.2004
(51) Int. Cl.: C12P 7/64

(54) **PROZESS ZUR KULTIVIERUNG VON MIKROORGANISMEN DER GATTUNG THRAUSTOCHYTRIEALES**
PROCESS FOR THE CULTIVATION OF MICROORGANISMS OF THE GENUS THRAUSTOCHYTRIALES
PROCEDE POUR CULTIVER DES MICRO-ORGANISMES DE L'ORDRE DES THRAUSTOCHYTRIALES

(30) Priorität: 10.11.2003 DE 10352837
(43) Veröffentlichungstag der Anmeldung: 02.08.2006
(73) Patentinhaber: Lonza Ltd, 4002 Basel (CH)
(72) Erfinder: LUY, Markus, 61267 Neu-Anspach (DE); RÜSING, Matthias, 50935 Köln (DE)
(74) Vertreter: Mai, Dörr, Besier
(86) Internationale Anmeldenummer: PCT/EP2004/012715
(87) Internationale Veröffentlichungsnummer: WO 2005/045050

(56) Entgegenhaltungen:
- EP-A- 0 113 183
- EP-A- 0 823 475
- WO-A-98/03671
- BAJPAI P K ET AL: "Optimization of production of docosahexaenoic acid (DHA) by Thraustochytrium aureum ATCC 34304" JOURNAL OF THE AMERICAN OIL CHEMISTS' SOCIETY, AMERICAN OIL CHEMISTS' SOCIETY. CHAMPAIGN, US, Bd. 68, Nr. 7, Juli 1991 (1991-07), Seiten 509-514, XP008023474 ISSN: 0003-021X
- PATENT ABSTRACTS OF JAPAN Bd. 013, Nr. 589 (C-670), 25. Dezember 1989 (1989-12-25) & JP 01 247079 A (NEC CORP), 2. Oktober 1989 (1989-10-02)
- PATENT ABSTRACTS OF JAPAN Bd. 1996, Nr. 12, 26. Dezember 1996 (1996-12-26) & JP 08 196288 A (KAIYO BIO TECHNOL KENKYUSHO:KK), 6. August 1996 (1996-08-06)

## Beschreibung

Verschiedene mehrfach-ungesättigte Fettsäuren (PUFAs für polyunsaturated fatty acids) und insbesondere omega-3 Fettsäuren (n3-Fettsäuren) sind essentielle Bestandteile der menschlichen Ernährung.

Allerdings ist bekannt, dass in den meisten Industrienationen die Versorgung mit n3-Fettsäuren mangelhaft ist. Dagegen ist der Gesamtfettanteil in der Ernährung, sowie die Zufuhr an gesättigten Fettsäuren und n6-Fettsäuren zu hoch. Dies beruht auf einer Veränderung unserer Nahrungszusammensetzung, die vor allem in den letzten ca. 150 Jahren stattgefunden hat und die mit dem Auftreten verschiedener chronischer Zivilisationskrankheiten, wie beispielsweise Herzkreislauferkrankungen - der Haupttodesursache in Industrienationen - korreliert wird (Simopoulos, A.P., 1999, Am. J. Clin. Nutr. 70, 560-569). Eine Vielzahl von Studien hat inzwischen gezeigt, dass durch die gezielte Erhöhung der Zufuhr von n3-Fettsäuren, insbesondere von Eicosapentaensäure (EPA) und von Docosahexaensäure (DHA), das Herzkreislaufrisiko signifikant reduziert werden kann (GISSI-Prevenzione Investigators (Gruppo Italiano per lo Studio della Sopravvivenza nell'Infarto miocardico), 1999, Dietary suplementation with n-3 polyunsaturated fatty acids and vitamin E after myocardial infarction: results of the GISSI-pevenzione trial., Lancet 354, 447-455; Burr et al., 1989, Effects of changes in fat, fish, and fibre intake on death and myocardial reinfarction: diet and reinfarction trial (DART). Lancet 2, 757-761): Dementsprechend wird von vielen verschieden Organisationen (WHO, FAO, AHA; ISSFAL, British Nutrition Foundation u.v.a.) empfohlen, die Zufuhr von n3-Fettsäuren signifikant zu erhöhen (Kris-Eherton et al., Fish Consumption, Fish Oil, Omega-3 Fatty Acids, and Cardiovascular Disease. Circulation 2002, 2747-2757).

Quellen für die Gewinnung von PUFAs und insbesondere n3-Fettsäuren sind vor allem marine Kaltwasserfische und daraus gewonnene Öle, aber auch marine Mikroorganismen, welche gegenüber Fischen den Vorteil haben, dass sie in Fermentern unter kostengünstigen und kontrollierten Bedingungen zur Produktion von PUFAs herangezogen werden können. Bei einer fermentativen Herstellung besteht keine Gefahr der Verunreinigungen, wie sie für Fische oder daraus gewonnene Fischöle oft beschrieben werden (Olsen SF. Int J Epidemiol. 2001: 1279-80). Außerdem lässt sich die Zusammensetzung der gewonnenen Öle durch Auswahl des Organismus und der Kulturbedingungen positiv beeinflussen und ist nicht saisonalen Schwankungen unterworfen, wie dies ebenfalls für Fisch und Fischprodukte beschrieben ist (Gamez-Meza et al. Lipids 1999:639-42).

Mikroorganismen, welche sich zur Gewinnung von n3-PUFA eignen, finden sich beispielsweise bei den Bakterien unter der Gattung *Vibrio* (z. B.: *Vibrio marinus*) oder unter den Dinoflagellaten (*Dinophyta*), dort insbesondere die Gattung *Crypthecodinium,* wie C. *cohnii* oder unter den Stramenopiles, wie die *Pinguiophyceae* wie z.B. *Glossomastix, Phaeomonas, Pinguiochrysis, Pinguiococcus* und *Polydochrysis.* Bevorzugte Mikroorganismen zur fermentativen Herstellung von PUFA gehören zu den Stramenopiles (oder *Labyrinthulomycota*), insbesondere zur Ordnung *Thraustochytriales,* (*Thraustchytriidea*) und dort wieder insbesondere zu den Gattungen *Japonochytrium, Schizochytrium, Thraustochytrium, Althornia, Labyrinthuloides, Aplanochytrium* und *Ulkenia.*

Es ist bekannt, dass einige der genannten Mikroorganismen zur industriellen Production von Fettsäuren verwendet werden können, entsprechende Verfahren wurden beschrieben. So offenbart die internationale Patentanmeldung WO 91/07498 A1 die Herstellung von PUFAs mit Organismen der Gattungen *Schizochytrium* und *Thraustochytrium.* WO 91/11918 Al offenbart die Herstellung von PUFAs mit *Crypthecodinium cohnii,* WO 96/33263 Al und die entsprechende europäische Patentanmeldung EP 0 823 475 A1 beschreiben die Herstellung von PUFAs mit Mikroorganismen der Gattung *Schizochytrium,* während die Patentanmeldung WO 98/03671 die Herstellung von PUFAs mit Mikroorganismen der Gattung *Ulkenia* offenbart.

Der natürliche Lebensraum der beschriebenen Mikroorganismen und insbesondere der *Labyrinthulomycota* sind marine Habitate. Herkömmlicherweise werden diese Mikroorganismen also in entsprechend salzhaltigen Medien kultiviert, wobei für die Zwecke der vorliegenden Erfindung der Salzgehalt von Meerwasser bei 32-35 g/l und einem Anteil von 90-95% an Natrium und Chlorid definiert wird. Typische Medien zur Kultivierung von marinen Mikroorganismen wie *Thraustochytrium* oder *Schizochytrium* basieren auf Seewasser (z.B. ATCC (American Type Culture Collection) 790 By+ Medium [Hefeextrakt 1.0 g, Peptone 1.0 g, D+-Glucose 5.0 g, Seewasser 1 L] ]). Es ist aber auch bekannt, dass Mikroorganismen der Ordnung *Thraustochytriales* bei sehr niedriger Salinität im Kulturmedium überleben können. Ihr Wachstum wird jedoch unterhalb einer Grenze von 7,5 - 15 g Salz/L, entsprechend einer Salinität von 7,5 - 15‰, als nur sehr gering und ohne Zwischenmaxima im niedrigen Salinitätsbereich beschrieben. Optimale Wachstumsraten werden nur oberhalb der oben angegebenen Salinitätsgrenze erzielt (Fan et al. Botanica Marina 45, 2002, S. 50-57).

Ein häufig auftretendes Problem fermentativer Prozesse sind starke pH-Schwankungen im Verlauf der Kultivierung als Folge des Auftretens von Stoffwechselprodukten und/oder des Verbrauchs von einzelnen Medienkomponenten. Dies gilt insbesondere für salzreiche Medien zur Fermentation mariner Mikroorganismen. Daher benötigen solche Fermentationen oft eine pH-Regulierung. Die Steuerung des pH-Wertes führt allerdings bei Fermentationen im Großmaßstab zu einem erheblichen Mehraufwand. Dabei werden zusätzliche Behältnisse für die Zugabe von Säuren und Laugen benötigt, die ansonsten anderweitig für die Zufütterung additiver Komponenten verwendet werden könnten. Weiterhin muß die Titration zur Regulierung des pH-Wertes technisch gesteuert werden. Im Zusammenhang mit der Fermentation von *Labyrinthulomycota* zur Gewinnung von PUFA im Produktionsmaßstab werden im Stand der Technik pH-Wert kontrollierte Kultivierungsmethoden eingesetzt.

Die Kontrolle des pH-Wertes über ansonsten in der Zellkultur übliche Puffersysteme weist jedoch Nachteile auf. So ist die Pufferkapazität von zum Beispiel TRIS, HEPES und MOPS aufgrund ihrer pKₐ-Werte über 7, im für die PUFA-Fermentation notwendigen pH-Bereich nicht ausreichend. TRIS ist weiterhin ein schlechter Puffer in pH-Bereichen unter 7,5, ein potentiell reaktives primäres Amin und kann an den unterschiedlichsten biologischen Reaktionen aktiv teilnehmen. Phosphatpuffer, ein ebenfalls häufig eingesetzter Puffer, hat die Eigenschaft bei Gegenwart von divalenten Kationen aus der Lösung auszufallen und ist ferner eine schlechte Wahl bei fermentativen Prozessen, die Phosphat benötigen bzw. verbrauchen. Acetatpuffer sind aufgrund ihres engen Pufferbereichs und durch die Tatsache, dass sie im Verlauf der Fermentation metabolisiert werden, ungeeignet. Darüber hinaus sind viele alternative Puffersysteme infolge hoher Kosten unwirtschaftlich.

In Anbetracht des Standes der Technik war es daher Aufgabe der vorliegenden Erfindung ein neues, einfaches und wirtschaftliches Kultivierungsverfahren für marine Mikroorganismen zur Verfügung zu stellen. Hierbei sollte eine starke Vereinfachung der Prozeßführung erreicht werden. Abgesehen von der Kosteneffizienz sollte das Verfahren die Herstellung hoch reiner PUFAs in hoher Ausbeute ermöglichen.

Gelöst werden diese sowie weitere, nicht explizit genannten Aufgaben, die jedoch aus den hierin einleitend diskutierten Zusammenhängen ohne weiteres ableitbar oder erschließbar sind, durch den Gegenstand, der in den Ansprüchen der vorliegenden Erfindung definiert ist. Die vorliegende Erfindung betrifft ein Verfahren zur Kultivierung von Mikroorganismen der Ordnung *Thraustochytriales,* bei welchem die Mikroorganismen in einem Fermentationsmedium kultiviert werden, das als das wesentliche Mittel zur pH-Wert-Stabilisierung CaCO₃ aufweist, das mit einem Gehalt von 3 - 15 g/l in dem Fermentationsmedium vorliegt, wobei CaCO₃ dann ein wesentliches Mittel zur pH-Wert-Stabilisierung ist, wenn der Betrag der Differenz der pH-Werte, die mit oder ohne Zusatz von Säuren - jeweils mit dem erfindungsgemäßen Zusatz von CaCO₃ - gemessen werden können, kleiner gleich 1 ist.

Bevorzugte PUFAs sind erfindungsgemäß DHA, DPA und EPA.

Insbesondere zeigen die in dem oben genannten Verfahren kultivierten Mikroorganismen eine Produktion von mehr als 10%, bevorzugt mehr als 14%, und ganz besonders bevorzugt mehr als 18% DHA pro Biotrockenmasse

Insbesondere zeigen die in dem oben genannten Verfahren kultivierten Mikroorganismen eine Produktion von mehr als 1%, bevorzugt mehr als 2%, und ganz besonders bevorzugt mehr als 3% DPA pro Biotrockenmasse.

Durch an die Kultivierung anschließende Isolation der PUFAs aus den Mikroorganismen Biomasse) und/oder dem Kulturmedium können die PUFAs in hoher Ausbeute und Reinheit gewonnen werden.

Weiterhin wird die Verwendung eines Kulturmediums umfassend als das wesentliche Mittel zur pH-Wert-Stabilisierung CaCO₃, das mit einem Gehalt von 3 - 15 g/l im Kulturmedium vorliegt, zur Kultivierung von Mikroorganismen der Ordnung *Thraustochyriales,* unter Schutz gestellt.

Dabei zeigen die Mikroorganismen unter erfindungsgemäßen Bedingungen eine Produktion von mehr als 25 Gew.-% Öl, bevorzugt von mehr als 35 Gew.-% Öl und ganz besonders bevorzugt von und als 45 Gew.-% ÖL pro Gewichtseinheit Biotrockenmasse.

Unter Öl wird erfindungsgemäß ein Anteil von mindestens 70 % Neutrallipiden und mindestens 2% Phospholipiden verstanden, was dem normalen, dem Fachmann bekannten Fettsäurespektrum der *Thraustochytriales* entspricht. Die Neutrallipide bestehen dabei aus mindestens 80% Triglyzeriden und anderen Verbindungen wie z.B Diacylglyzeride, Sterole usw. Weiterhin besteht der Gewichtsanteil der Triglyzeride aus etwa 95% Fettsäuren und 5% Glyzerin.

Die Möglichkeit einen marinen Mikroorganismus für die Produktion von PUFA ohne externe pH-Wert Regulierung fermentieren zu können, insbesondere unter Bedingungen, die ein rasches Wachstum unter hohem Glukoseverbrauch ermöglichen, war völlig überraschend. Gerade unter solchen Bedingungen kommt es bei der Fermentation von marinen Mikroorganismen ohne entsprechende pH-Kontrolle sehr schnell zu einer Ansäuerung des Mediums, die zum Einstellen des Wachstums führt (siehe Beispiel 1 und Wen, Z.-Y. and Chen, F., 2003, Biotechnology Advances 21, 273-294).

Das erfindungsgemäße Verfahren kommt überraschenderweise ohne den Zusatz sonstiger pH-Wert stabilisierender Mittel aus. Unter pH-Wert stabilisierenden Mitteln wird erfindungsgemäß sowohl der in Abhängigkeit von dem sich während der Kultur einstellenden pH-Wert geregelte Zusatz von Säure oder Base aus Zusatztanks als auch die Verwendung von Puffersystemen im Medium selbst verstanden, obwohl den Erfindern wirtschaftlich nutzbare Kultivierungsverfahren unter Verwendung von Puffersystemen wie beispielsweise TRIS oder Phosphat-Puffer nicht bekannt sind.

Erfindungsgemäß ist CaCO₃ das wesentliche Mittel zur pH-Wert Stabilisierung. Trotzdem kann es sein, dass unter bestimmten Bedingungen der Zusatz von Säure oder Base zur Kultur zur Einstellung eines pH-Wertes nötig sein kann. Ein solcher Zusatz ist von der Erfindung mitumfasst, solange CaCO₃ das wesentliche Mittel zur pH-Wert Stabilisierung bleibt. Fällt beispielsweise der pH-Wert während der Kultivierung aufgrund besonders schnellen Wachstums der Mikroorganismen unter einen bestimmten Zielwert, so kann dieser Zielwert kurzfristig durch Zugabe von Säure oder Base eingestellt werden, ohne dass dieser Zusatz das wesentliche Mittel zur pH-Wert Regulierung ist.

Die Begriffe pH-Wert Regulierung und pH-Wert Steuerung bzw. pH-Wert Stabilisierung werden erfindungsgemäß synonym verwendet.

Wesentliches Mittel bleibt CaCO₃, wenn der Betrag der Differenz der pH-Werte, die mit oder ohne Zusatz von Säuren - jeweils mit dem erfindungsgemäßen Zusatz von CaCO₃- gemessen werden können, kleiner gleich 1 ist, bevorzugt kleiner gleich 0,75, besonders bevorzugt kleiner gleich 0,5, ganz besonders bevorzugt kleiner gleich 0,2 und am meisten bevorzugt kleiner gleich 0,1 ist. Ein solcher Zusatz von Säuren und/oder Basen wird als erfindungsgemäß geringfügiger Zusatz von Säuren und/oder Basen verstanden, der von der Erfindung mitumfasst ist.

Bevorzugt sind Kultivierungssysteme, die gänzlich ohne Einsatz von Säure- und/oder Basezusatz auskommen.

Darüber hinaus sind viele alternative Puffersysteme gegenüber der Verwendung von Calciumcarbonat, infolge höherer Kosten, unwirtschaftlich.

Die hohe Wirksamkeit von Calciumcarbonat als Puffer für die Kultivierung von Mikroorganismen der Ordnung *Thraustochytriales* ist überraschend, da entstehendes Kohlendioxid nur eine begrenzte Löslichkeit in Wasser besitzt und dadurch die Pufferkapazität während der Fermentation abnimmt.

Erstaunlicherweise war nicht nur die Fermentation bis zum vollständigen Verbrauch von Glukose möglich, es erhöhte sich darüber hinaus auch der Anteil der PUFA in der Biomasse signifikant bei Einsatz des erfindungsgemäßen Calciumcarbonat stabilisierten Mediums. Noch überraschender ist, dass die Glukoseverwertung und damit verbunden die PUFA-Produktion beschleunigt wird und so zu einer erhöhten Raum-Zeit-Ausbeute führt.

Bis zur vorliegenden Erfindung gab es keinen bekannten Fermentationsprozess zur Produktion von n3-Fettsäuren in Mikroorganismen aus der Ordnung *Thraustochytriales* unter Verwendung eines mit Calciumcarbonat pH-stabilisierten Mediums, wobei auf weitere pH-Wert stabilisierende Mittel verzichtet werden konnte.

PUFAs sind erfindungsgemäß mehrfach ungesättigte langkettige Fettsäuren mit einer Kettenlänge >C12 mit mindestens zwei Doppelbindungen. Nach dem erfindungsgemäßen Verfahren herstellbare PUFAs sind insbesondere n3-Fettsäuren und n6-Fettsäuren.

Unter n3-Fettsäuren (omega-3 Fettsäure, ω3 Fettsäuren) werden mehrfach ungesättigte langkettige Fettsäuren mit einer Kettenlänge >C12 mit mindestens zwei oder mehr Doppelbindungen verstanden, wobei die erste der Doppelbindungen zwischen den Kohlenstoffatomen C3 und C4 ausgehend vom Alkylende konstituiert ist. Dementsprechend liegt bei n6-Fettsäuren die erste Doppelbindung zwischen den Kohlenstoffatomen C6 und C7 ausgehend vom Alkylende.

Für die Produktion der PUFAs nach dem erfindungsgemäßen Verfahren kommen Mikroorganismen der Ordnung *Thraustochytriales* (*Thraustchytriidea*) in Frage (Lewis, T.E., Nichols, P.D., McMeekin, T.A., The Biotechnological Potential of Thraustochytrids, Marine Biotechnology, 1999, S. 580-587 und Porter, D. Phylum Labyrinthulomycota in Handbook of protoctista: the structure, cultivation, habitats, and life histories of the eukaryotic microorganisms and their descendants exclusive of animals, plants, and fungi: a guide to the algae, ciliates, foraminifera, sprorozoa, water molds, and other protoctists. Editors: Margulis, L, Corliss, J.O., Melkonian, M. and Chapman, D.J., editorial coordinator, McKhann, H.I., Jones and Bartlett Publishers, ISBN 0-86720-052-9 1990, S. 388-398). Besonders bevorzugt sind Mikroorganismen der Gattungen *Japonochytrium, Schizochytrium, Thraustochytrium Althornia, Labyrinthuloides, Aplanochytrium* und *Ulkenia.* Ganz besonders bevorzugt sind hiervon *Schizochytrium, Thraustochytrium* und *Ulkenia.* Insbesondere bevorzugt sind: *Japonochytrium* sp. ATCC 28207, *Thraustochytrium aureum* (insbesondere ATCC 28211 bzw. ATTC 34304), *Thraustochytrium roseum* ATCC 28210 *Thraustochytrium* sp. ATCC 20890, ATTC 20891, ATTC 20892 und ATTC 26185, *Schizochytrium aggregatum* ATTC 28209, *Schizochytrium* sp. ATCC 20888 und ATTC 20889, *Schizochytrium* SR21, sowie *Ulkenia* spec. SAM 2179 und SAM 2180.

Für das erfindungsgemäße Verfahren geeignete Mikroorganismen sind sowohl Wildtyp Formen als auch Mutanten und daraus abgeleitete Stämme sowie rekombinante Stämme der entsprechenden Organismen. In besonderem Maße umfasst die vorliegende Erfindung Mutanten oder rekombinante Stämme zur Produktionssteigerung von PUFA.

Die Mikroorganismen werden durch Animpfen eines flüssigen oder eines festen Mediums mit einer Vorkultur dieser Organismen kultiviert.

Für Mikroorganismen der Ordnung *Thraustochytriales* geeignete Kulturtechniken sind dem Fachmann gut bekannt. Typischerweise, aber nicht ausschließlich, wird die Kultur mittels wässriger Fermentation in einem entsprechenden Behältnis durchgeführt. Beispiele für typische Behältnisse für eine derartige Fermentation umfassen Schüttelkolben oder Bioreaktoren, wie beispielsweise STRs (stirred tank reactors) oder Blasensäulen. Die Kultur wird typischerweise bei Temperaturen zwischen 10°C und 40°C durchgeführt, bevorzugterweise zwischen 20°C und 35 °C, besonders bevorzugt zwischen 25°C und 30°C, ganz besonders bevorzugt zwischen 27°C und 29°C und insbesondere bei 28 ±0,5°C.

Im Rahmen der vorliegenden Erfindung entspricht der Calciumcarbonatgehalt des pH-stabilisierten Mediums einem Wert im Bereich von 3g/L bis 15g/L, bevorzugt von 4g/L bis 12g/L und besonders bevorzugt von 5g/L bis 10g/L. Ganz besonders bevorzugt ist ein Calciumcarbonatgehalt von 7,5 ±0,5g/L.

Das pH-Wert stabilisierte Medium umfasst ferner bevorzugterweise eine oder mehrere Kohlenstoffquellen, sowie eine oder mehrere Stickstoffquellen. Dem Fachmann sind für die Kultivierung von Mikroorganismen der Ordnung *Thraustochytriales* als Kohlenstoff- und Stickstoffquellen verwendbare Substanzen gut bekannt.

Verwendbare Kohlenstoffquellen sind beispielsweise Kohlenhydrate wie Glukose, Fruktose Xylose, Saccharose, Maltose, lösliche Stärke, Fucose, Glucosamin, Dextran, Glutaminsäure, Melasse, Glyzerin oder Mannitol oder auch Fette und Öle oder pflanzliche Hydrolysate.

Einsetzbare natürliche Stickstoffquellen sind beispielsweise Pepton, Hefeextrakt, Malzextrakt, Fleischextrakt, Casaminosäuren, Maisquellwasser oder Sojabohnen, einsetzbare organische Stickstoffquellen sind beispielsweise Glutamat und Harnstoff, aber auch anorganische Stickstoffquellen wie zum Beispiel Ammoniumacetat, Ammoniumhydrogencarbonat, Ammoniumsulfat oder Ammoniumnitrat können als Stickstoffquelle verwendet werden.

Das pH-Wert stabilisierte Medium kann neben Calciumcarbonat alle weiteren dem Fachmann für die Kultivierung von Mikroorganismen der Ordnung *Thraustochytriales* förderlichen Bestandteile enthalten, insbesondere anorganische Salze von beispielsweise Ca, Mg, Na, K, Fe, Ni, Co, Cu, Mn, Mo oder Zn. Beispielhaft seien Phosphate wie Kaliumdihydrogenphosphat oder Chloride wie Magnesiumchlorid, Sulfate, wie Ammoniumsulfat, Magnesiumsulfat, Eisensulfat oder Natriumsulfat genannt. Weitere verwendbare anorganische Salze sind beispielsweise Halogenide, wie Kaliumbromid oder Kaliumjodid und ebenso weitere Carbonate wie zum Beispiel Natriumhydrogencarbonat.

Ggf. kann das Medium zusätzliche Makro- oder Mikronährstoffe umfassen, wie Aminosäuren, Purine, Pyrimidine, Corn Steep Liquor (Maisquellwasser), Proteinhydrolysate, Vitamine (wasserlöslich und/oder wasserunlöslich) und andere dem Fachmann gut bekannte Medienbestandteile. Antischaummittel können zugegeben werden, falls notwendig. Das Medium kann komplexe Bestandteile enthalten oder chemisch definiert sein.

Die Menge der einzelnen Komponenten kann variieren, solange kein negativer Effekt auf das Wachstum oder die Produktivität der Mikroorganismen vorliegt. Der Fachmann kann die Zusammensetzung entsprechend den Bedürfnissen des Mikroorganismus im Einzelfall leicht ermitteln. Im Allgemeinen wird die Kohlenstoffquelle in einer Konzentration bis 300 g/l und die Stickstoffquelle in einer Konzentration von 1 bis 30 g/l zugegeben. Vorzugsweise wird der Stickstoffgehalt in Abhängigkeit vom Kohlenstoffgehalt des Mediums gestellt.

Ein pH-Wert stabilisiertes Medium kann Glukose, Hefeextrakt, Maisquellwasser (Corn Steep Liqour [CSL]), Magnesiumchlorid, Calciumcarbonat, Calciumchlorid, Natriumsulfat und Kaliumphosphat umfassen.

Der pH-Wert des Mediums wird vor Beginn der Fermentation auf einen Bereich von 3 bis 10, bevorzugt 4 bis 8, besonders bevorzugt 5 bis 7 und ganz besonders bevorzugt etwa 6 durch Zugabe einer entsprechenden Säure oder Lauge eingestellt.

Anschließend wird das Medium sterilisiert. Techniken zur Sterilisation von Medien sind dem Fachmann gut bekannt, beispielhaft seien Autoklavieren und Sterilfiltration genannt.

Die Kultivierung kann in Batch-, Fed-Batch- oder in kontinuierlicher Weise erfolgen, wie es dem Fachmann allgemein bekannt ist.

Eine Batch- oder Fed-Batch-Kultivierung erfolgt üblicherweise über 1 bis 12 Tage, bevorzugt 1-10 Tage, besonders bevorzugt für 3-9 Tage.

Die Medienbestandteile können dem Medium einzeln oder vermischt zugegeben werden, auch eine vorgefertigte Mischung ist denkbar. Die Bestandteile, insbesondere die Kohlenstoff- und Stickstoffquelle(n) oder bestimmte Mediumszusätze können vor oder während der Kultivierung zugegeben werden. Die Zugabe kann einmal oder mehrmals wiederholt oder auch kontinuierlich erfolgen.

Die produzierten PUFA liegen im Allgemeinen in Form von Neutralfetten zum Beispiel als Triacylglyzeride oder polare Lipide wie zum Beispiel Phosphatidylcholin, Phosphatidylethanolamin oder Phosphatidylinositol vor.

Für die Zwecke der vorliegenden Erfindung werden unter den Begriffen PUFA, n3-Fettsäure oder n3-Wirkstoffe alle möglichen Formen verstanden, in denen die entsprechenden Fettsäuren vorliegen können, d. h. sowohl als freie Fettsäuren, Ester, Triglyceride, Phospholipide oder andere Derivate. All diese Substanzen werden im Folgenden zusammengefasst und die Begriffe werden synonym verwendet. Im weiteren können die PUFAs durch chemische oder biokatalytische Umesterung beispielsweise mit Hilfe geeigneter Enzyme (Lipasen) umgesetzt und angereichert werden, vor oder nach der Isolierung aus der Kultur.

Die Isolierung von PUFAs aus den fermentierten Mikroorganismen bzw. dem Medium und die Analyse des Fettsäurespektrums erfolgt nach für den Fachmann bekannten und üblichen Verfahren (Wanasundara, U.N., Wanasundara, J., Shahidi, F., Omega-3 fatty acid concentrates: a review of production technologies, Seafoods - Quality, Technology and Nutraceutical Applications, 2002, S. 157-174).

Nachfolgend wird das dem erfindungsgemäßen Verfahren zugrunde liegende pH-stabilisierte Fermentationsmedium anhand einiger Beispiele und Vergleichsbeispiele beschriben. Das Fermentationsmedium sowie die Erfindung sind jedoch nicht auf diese Beispiele beschränkt.

### Beispiel 1: Fennentation von Ulkenia spec. Stamm SAM 2179 zur Produktion von PUFA in verschiedenen, ausschließlich durch unterschiedliche Mengen von CaCO₃ pH-Wert stabilisierten Kulturmedien.

*Ulkenia* spec. Stamm SAM 2179 wurde in 300ml Erlenmeyerkolben mit einer Schikane in 50mL Medium kultiviert.

Medienzusammensetzung:
Fermentationsmedium:

| | | |
|---|---|---|
| Glucose | | 150g/L |
| Maisquellwasser | | 3,75 g/L |
| KH₂PO₄ | | 3g/L |
| Na₂SO₄ | | 1g/L |
| MgCl₂x6H₂O | 1g/L | |
| CaCl₂x2H₂O | 0,3g/L | |
| (NH₄)₂SO₄ | 5g/L | |

Zusatz CaCO₃ je 50mL Kolben:

| | |
|---|---|
| Medium 1.1 | 0g/L (Vergleich) |
| Medium 1.2 | 1g/L (Vergleich) |
| Medium 1.3 | 2g/L (Vergleich) |
| Medium 1.4 | 5g/L |
| Medium 1.5 | 10g/L |

pH-Wert mit NaOH auf 6,0 einstellen und autoklavieren

Kulturbedingungen:

| | | |
|---|---|---|
| Temperatur (°C): | | 28 |
| Schüttelrate (rpm): | 150 | |

Zellernte erfolgte nach 96h Kultivierung durch Zentrifugation. Anschließend wurden die Zellen gefriergetrocknet und die Biotrockenmasse bestimmt. Aufschluß der Zellen und Bestimmung der Fettsäuren erfolgte durch Hitzebehandlung für 2 Stunden in 10%iger methanolischer Salzsäure bei 60°C (unter Rühren). Die Ester wurden dann im Gaschromatographen zur Bestimmung der Fettsäurezusammensetzung analysiert.

**Tabelle 1 : Fermentationsparameter in Abhängigkeit von der Calciumcarbonatkonzentration**

| | **CaCO₃** | **Glukose-verbrauch** | **pH-Wert** | **BTM** | **DHA-Area** | **DHA/BTM** | **DHA** | **DHA-RZA** |
|---|---|---|---|---|---|---|---|---|
| | **(g/L)** | **(g/L)** | | **(g/L)** | **(%)** | **(%)** | **(g/L)** | **(g/Lxd)** |
| **Medium 1.1** | 0 | 43,0 | 1,85 | 22,72 | 48,3 | 3,35 | 0,76 | 0,19 |
| **Medium 1.2** | 1 | 59,0 | 2,31 | 30,32 | 44,0 | 4,91 | 1,49 | 0,37 |
| **Medium 1.3** | 2 | 81,7 | 2,84 | 38,58 | 43,9 | 9,90 | 3,82 | 0,96 |
| **Medium 1.4** | 5 | 108,4 | 5,02 | 52,99 | 44,8 | 15,72 | 8,33 | 2,08 |
| **Medium 1.5** | 10 | 111,9 | 4,78 | 52,32 | 45,5 | 13,23 | 6,92 | 1,73 |

| | | | | | | | | |
|---|---|---|---|---|---|---|---|---|
| BTM: Biotrockenmasse; DHA/BTM: Gew.-% DHA (Docosahexaensäure) pro Gewichtseinheit BTM; g/lxd Raum-Zeit-Ausbeute in Gramm pro Liter pro Tag; RZA: Raum-Zeit-Ausbeute; DHA-Area (%) Anteil DHA im Fettsäurespektrum | | | | | | | | |

Die Fermentation von *Ulkenia* spec. SAM 2179 in Fermentationsmedium ohne ausreichende pH-Stabilisierung führt im Verlauf der Fermentation zu einer Verlangsamung des Glukoseverbrauchs und zur Einstellung des Wachstums, infolge eines starken Absinkens des pH-Wertes (siehe Medium 1.1. - 1.3.). Erst größere Mengen von CaCO₃-Puffer führen zu einer Stabilisierung des pH-Wertes während der Kultur (Medium 1.4 und 1.5). Dabei kommt es mit zunehmender CaCO₃-Konzentration auch zu einem erhöhten Glukoseverbrauch während der Kultur. Infolge der pH-Wert Stabilisierung und dem damit zusammenhängenden erhöhten Glukoseverbrauch werden höhere Biomassewerte und daraus resultierend auch höhere DHA-Raum-Zeit-Ausbeuten, die unter den oben angegebenen Experimentalbedingungen bei etwa 2g/Lxd liegen, erzielt.

### Beispiel 2: Fermentation von Ulkenia spec. Stamm SAM 2179 zur Produktion von PUFA in verschiedenen, ausschließlich durch unterschiedliche Mengen von CaCO₃ pH-Wert stabilisierten, Kulturmedien bis zur Glukoselimitierung.

*Ulkenia* spec. Stamm SAM 2179 wurde in 300ml Erlenmeyerkolben mit einer Schikane in 50mL Medium bis zum vollständigen Glukoseverbrauch kultiviert.

Medienzusammensetzung:
Fermentationsmedium:

| | | |
|---|---|---|
| Glucose | | 150g/L |
| Maisquell -wasser | | 3,75g/L |
| KH₂PO₄ | | 3g/L |
| Na₂SO₄ | | 1g/L |
| MgCl₂x6H₂O | 1g/L | |
| CaCl₂x2H₂O | 0,3g/L | |
| (NH₄)₂SO₄ | 5g/L | |

Zusatz CaCO₃ je 50mL Kolben:

| | |
|---|---|
| Medium 1.4 | 5g/L |
| Medium 1.5 | 10g/L |

pH-Wert mit NaOH auf 6,0 einstellen und autoklavieren

Kulturbedingungen:

| | | |
|---|---|---|
| Temperatur (°C): | | 28 |
| Schüttelrate (rpm): | 150 | |

Die Zellemte erfolgte nach 144,5h Kultivierung durch Zentrifugation. Anschließend wurden die Zellen gefriergetrocknet und die Biotrockenmasse bestimmt. Aufschluß der Zellen und Bestimmung der Fettsäuren erfolgte durch Hitzebehandlung für 2 Stunden in 10%iger methanolischer Salzsäure bei 60°C (unter Rühren). Die Ester wurden dann im Gaschromatographen zur Bestimmung der Fettsäurezusammensetzung analysiert.

**Tabelle 2: Fermentationsparameter nach Glukoselimitierung**

| | **CaCO₃** | **Glukose-verbrauch** | **pH-Wert** | **BTM** | **DHA-Area** | **DHA/BTM** | **DHA** | **DHA-RZA** |
|---|---|---|---|---|---|---|---|---|
| | **(g/L)** | **(g/L)** | | **(g/L)** | **(%)** | **(%)** | **(g/L)** | **(g/Lxd)** |
| **Medium 1.4** | 5 | 150,0 | 6,49 | 58,52 | 47,7 | 22,20 | 12,99 | 2,14 |
| **Medium 1.5** | 10 | 150,0 | 6,58 | 64,65 | 46,7 | 20,54 | 13,28 | 2,19 |

Die Fermentation von *Ulkenia* spec SAM 2179 in mit 5g/L bzw. 10g/L CaCO₃ gepufferten Medium ermöglicht eine Kultivierung bis zur Glukoselimitierung, ohne starkes Absinken des pH-Wertes. Die dabei erreichbare Biomasse und der Anteil von DHA pro Biomasse sind, mit etwa 58-64g/L Biomasse und 20-22% DHA/BTM, entsprechend dem vollständigen Glukoseverbrauch sehr hoch. Hierbei zeigt sich, dass die höhere CaCO₃-Konzentration (10g/L) zu einer größeren Biomasse führt, der Anteil der essentiellen PUFA DHA pro Biomasse gegenüber der niedrigeren Konzentration CaCO₃ (5g/L) jedoch etwas abnimmt. Die dabei erzielte Raum-Zeit-Ausbeute an DHA bleibt allerdings bei beiden Konzentrationen etwa gleich.

### Beispiel 3: Kultivierung von Ulkenia spec. Stamm SAM 2179, zur Produktion von PUFA, unter optimierten Fermentationsbedingungen.

*Ulkenia* spec Stamm SAM 2179 wurde in 300ml Erlenmeyerkolben mit einer Schikane in 50mL Medium bis zum vollständigen Glukoseverbrauch kultiviert. Die Optimierung der Fermentation resultierte aus einer CaCO₃-Konzentration von 7,5g/L und einer veränderten Vorkultur. Für die Vorkultur wurde anstelle einer Standkultur in DH1-Medium eine Schüttelkultur mit gleichem Medium verwendet (48h, 150rpm und 28°C).

Medienzusammensetzung:
Vorkulturmedium: DH1-Medium

| | |
|---|---|
| Glukose-Monohydrat (g/l): | 56,25 |
| Hefeextrakt (g/l): | 12,5 [Difco] |
| Tropic Marin (g/l): | 16,65 [Dr. Biener GmbH, Wartenberg, Germany] pH-Wert mit HCl auf 6,0 eingestellt |

Fermentationsmedium:

| | | |
|---|---|---|
| Glucose | | 150g/L |
| Maisquell -wasser | | 3,75g/L |
| KH₂PO₄ | | 3g/L |
| Na₂SO₄ | | 1g/L |
| MgCl₂x6H₂O | 1g/L | |
| CaCl₂x2H₂O | 0,3g/L | |
| (NH₄)₂SO₄ | 5g/L | |

Zusatz CaCO₃ je 50mL Kolben: Medium 1.6 7,5g/L
pH-Wert mit NaOH auf 6,0 einstellen und autoklavieren

Kulturbedingungen:

| | |
|---|---|
| Temperatur (°C): | 28 |
| Schüttelrate (rpm): | 150 |

Die Zellernte erfolgte nach 99,75h Kultivierung durch Zentrifugation. Anschließend wurden die Zellen gefriergetrocknet und die Biotrockenmasse bestimmt. Aufschluß der Zellen und Bestimmung der Fettsäuren erfolgte durch Hitzebehandlung für 2 Stunden in 10%iger methanolischer Salzsäure bei 60°C (unter Rühren). Die Ester wurden dann im Gaschromatographen zur Bestimmung der Fettsäurezusammensetzung analysiert.

**Tabelle 3: Fermentationsparameter unter optimierten Pufferbedingungen**

| | **CaCO₃** | **Glukose-verbrauch** | **pH-Wert** | **BTM** | **DHA-Area** | **DHA/BTM** | **DHA** | **DHA-RZA** |
|---|---|---|---|---|---|---|---|---|
| | **(g/L)** | **(g/L)** | | **(g/L)** | **(%)** | **(%)** | **(g/L)** | **(g/Lxd)** |
| **Medium 1.6** | 7,5 | 150,0 | 6,76 | 63,84 | 44,1 | 23,06 | 14,72 | 3,54 |

Durch den Einsatz optimierter Fermentationsbedingungen, bei denen *Ulkenia* spec. SAM 2179 zunächst 48h bei 28°C und 150rpm in DH1-Medium kultiviert wurde, konnte die DHA-Raum-Zeit-Ausbeute auf mehr als 3,5g/Lxd erheblich verbessert werden. Dies resultiert in erster Linie aus einem schnelleren Wachstum, wobei die Glukoselimitierung in weniger als 100h erreicht wurde. Der Einsatz von 7,5g/L CaCO₃ im Fermentationsmedium ermöglicht eine für die optimierte Kultivierung notwendige pH-Stabilisierung. Der Einsatz von 7,5g/L CaCO₃ ergab sich aus den Ergebnissen von Beispiel 2, bei denen 10g/L CaCO₃ höhere Biomassewerte ergaben, 5g/L CaCO₃ hingegen zu besseren DHA-Werten führten. Somit wurde eine für die Produktion von DHA optimale CaCO₃-Konzentration (d.h. möglichst hohe Biomasse bei möglichst hohem DHA-Gehalt) zwischen 5 und 10g/L CaCO₃ vermutet. Neben der Stabilisierung des pH-Wertes führt der Einsatz des beschriebenen Fermentationsmediums zu einer überraschend hohen DHA Raum-Zeit-Ausbeute von über 3g/Lxd zum Zeitpunkt der Glukoselimitierung. Hierbei wurden ähnliche Biomassewerte wie in Beispiel 2 erreicht, darüber hinaus waren aber der Anteil an DHA pro Biotrockenmasse und die erzielten DHA-Mengen (mehr als 10%) größer.

### Beispiel 4: Fermentation von Ulkenia spec. Stamm SAM 2179 zur Produktion von PUFA in Kulturmedium mit und ohne pH-Stabilisierung durch CaCO₃.

*Ulkenia* spec. Stamm SAM 2179 wurde jeweils in einem 5L-Fermenter bis zum vollständigen Glukoseverbrauch kultiviert.

Medienzusammensetzung:
Fermentationsmedium:

| | | |
|---|---|---|
| Glucose | 150g/L | |
| Maisquell -wasser | | 3,75g/L |
| KH₂PO₄ | | 3 g/L |
| Na₂SO₄ | | 1g/L |
| MgCl₂x6H₂O | 1g/L | |
| CaCl₂x2H₂O | 0,3g/L | |
| (NH₄)₂SO₄ | 5g/L | |

| | |
|---|---|
| Für die Fermentation mit pH Steuerung: | pH-Wert mit H₃PO₄ auf 4,0 einstellen und autoklavieren |
| Für die Fermentation ohne pH-Steuerung: | pH-Wert mit NaOH auf 6,0 einstellen und autoklavieren sowie Zusatz von 7,5g/L CaCO₃ |

Kulturbedingungen:

| | |
|---|---|
| Temperatur (°C): | 28 |
| Belüftung: | 0,8vvm |

Fermentation mit und ohne pH-Steuerung

**Tabelle 4: Fermentationsparameter mit und ohne pH-Steuerung**

| **pH-Steuerung** | **CaCO₃** | **Glukose-verbrauch** | **Zeitpunkt Glukose-verbrauch** | **BTM** | **DHA-Area** | **DHA/BTM** | **DHA** | **DHA-RZA** |
|---|---|---|---|---|---|---|---|---|
| | **(g/L)** | **(g/L)** | **(h)** | **(g/L)** | **(%)** | **(%)** | **(g/L)** | **(g/Lxd)** |
| + | 0 | 150,0 | 162 | 66,9 | 47,2 | 25,9 | 17,35 | 2,5 |
| - | 7,5 | 150,0 | 150 | 67,8 | 46,7 | 26,9 | 18,30 | 2,9 |

Der Einsatz des durch CaCO₃ -stabilisierten Fermentationsmediums ermöglicht auch im 5L-Fermentationsmaßstab eine Kultivierung von Ulkenia spec SAM 2179 bis zur Glukoselimitierung, ohne pH-Steuerung. Der Einsatz von CaCO₃ in ausreichender Menge führt zu einem schnelleren Wachstum als Folge eines beschleunigten Glukoseverbrauchs. Darüber hinaus werden höhere Biomassen erzielt. Weiterhin werden in diesem Zusammenhang während der Fermentation ein erhöhter DHA-Anteil pro Biomasse und größere Mengen DHA erzielt. Dies führt zu einer nicht unerheblichen Steigerung der DHA-Raum-Zeit-Ausbeute bei CaCO₃-gepufferter gegenüber pH-gesteuerter Fermentation von mehr als 15%.

### Beispiel 5: Fermentation von Schizochytrium spec. SR21 zur Produktion von PUFA in Fermentationsmedium 1.6 stabilisiert durch 7,5g/L CaCO₃

*Schizochytrium* spec. Stamm SR21 wurde in 300ml Erlenmeyerkolben mit einer Schikane in 50mL Medium bis zum vollständigen Glukoseverbrauch kultiviert.

Medienzusammensetzung:
Vorkulturmedium: GY-Medium

| | |
|---|---|
| Glukose (g/l): | 30,0 |
| Hefeextrakt (g/l): | 10,0 [Difco] |
| Tropic Marin (g/l): | 16,65 [Dr.Biener GmbH, Wartenberg, Germany] pH-Wert mit HCl auf 6,0 eingestellt |

Fermentationsmedium:

| | | |
|---|---|---|
| Glucose | | 150g/L |
| Maisquell -wasser | | 3,75g/L |
| KH₂PO₄ | | 3g/L |
| Na₂SO₄ | | 1g/L |
| MgCl₂x6H₂O | 1g/L | |
| CaCl₂x2H₂O | 0,3g/L | |

| | | |
|---|---|---|
| (NH₄)₂SO₄ | 5g/L | |

Zusatz CaCO₃ je 50mL Kolben: Medium 1.6 7,5g/L
pH-Wert mit NaOH auf 6,0 einstellen und autoklavieren
Kulturbedingungen:

| | | |
|---|---|---|
| Temperatur (°C): | | 28 |
| Schüttelrate (rpm): | 150 | |

Die Zellemte erfolgte nach 96h Kultivierung durch Zentrifugation. Anschließend wurden die Zellen gefriergetrocknet und die Biotrockenmasse bestimmt. Aufschluß der Zellen und Bestimmung der Fettsäuren erfolgte durch Hitzebehandlung für 2 Stunden in 10%iger methanolischer Salzsäure bei 60°C (unter Rühren). Die Ester wurden dann im Gaschromatographen zur Bestimmung der Fettsäurezusammensetzung analysiert.

**Tabelle 5: Fermentationsparameter unter optimierten Pufferbedingungen**

| | **CaCO₃** | **Glukose-verbrauch** | **pH-Wert** | **BTM** | **DHA-Area** | **DHA/BTM** | **DHA** | **DHA-RZA** |
|---|---|---|---|---|---|---|---|---|
| | **(g/L)** | **(g/L)** | | **(g/L)** | **(%)** | **(%)** | **(g/L)** | **(g/L x d)** |
| **SR 21** | 7,5 | 150,0 | 7,35 | 66,12 | 34,4 | 15,28 | 10,10 | 2,52 |

Die in der Erfindung beschriebene Verwendung des durch CaCO₃ pH-stabilisierten Mediums führt auch bei anderen Organismen der *Labyrinthulomycota* zu einer Optimierung der Produktion von PUFA. So kann beispielsweise der Mikroorganismus *Schizochytrium* spec. Stamm SR21 in dem beschriebenen Medium fermentiert werden. Die Raum-Zeit-Ausbeute der essentiellen PUFA, DHA in SR 21 ist etwas niedriger als in Ulkenia sp. SAM 2179 (siehe Beispiel 3), liegt aber bezogen auf die essentielle n-3 PUFA DHA über 15% (w/w) der gesamten Biotrockenmasse. Dieses Beispiel zeigt, dass die der Erfindung zugrunde liegende Verwendung des optimierten pH-stabilisierten Mediums eine Fermentation ohne pH-Steuerung zur Production von PUFAs auch in weiteren Mitgliedern der *Labyrinthulomycota* ermöglicht.

## Patentansprüche

1. Verfahren zur Kultivierung von Mikroorganismen der Ordnung Thraustochytriales, **dadurch gekennzeichnet, dass** die Mikroorganismen in einem Fermentationsmedium kultiviert werden, dass als das wesentliche Mittel zur pH-Wert-Stabilisierung CaCO₃ aufweist, das mit einem Gehalt von 3 - 15 g/l in dem Fermentationsmedium vorliegt, wobei CaCO₃ dann ein wesentliches Mittel zur pH-Wert-Stabilisierung ist, wenn der Betrag der Differenz der pH-Werte, die mit oder ohne Zusatz von Säuren - jeweils mit dem erfindungsgemäßen Zusatz von CaCO₃ - gemessen werden können, kleiner gleich 1 ist.

2. Verfahren nach Anspruch 1, wobei die Mikroorganismen eine Produktion von mehr als 25, bevorzugt von mehr als 35 und ganz besonders bevorzugt von mehr als 45 Gew.-% Öl pro Gewichtseinheit Biotrockenmasse hervorbringen.

3. Verfahren nach Anspruch 1, wobei die Mikroorganismen eine Produktion von mehr als 10, bevorzugt von mehr als 14, besonders bevorzugt von mehr als 18, und ganz besonders bevorzugt von mehr als 22 Gew.-% Docosahexaensäure (DHA) pro Gewichtseinheit Biotrockenmasse hervorbringen.

4. Verfahren nach einem der vorstehenden Ansprüche, wobei die Mikroorganismen eine Produktion von mehr als 1%, bevorzugt mehr als 2%, besonders bevorzugt mehr als 3% und ganz besonders bevorzugt von mehr als 4% Docosapentaensäure (DPA) pro Biotrockenmasse hervorbringen.

5. Verfahren nach Anspruch 1 oder 2, wobei dem Medium 3g/L bis 15g/L, bevorzugt 4g/L bis 12 g/L, besonders bevorzugt 5g/L bis 10g/L und ganz besonders bevorzugt 7,5 ±0,5 g/L CaCO₃ zugesetzt werden.

6. Verfahren nach einem der Ansprüche 1 bis 5 **dadurch gekennzeichnet, dass** das Medium Glukose, Maisquellwasser, Magnesiumchlorid, Calciumchlorid, Calciumcarbonat, Natriumsulfat, Ammoniumsulfat und Kaliumhydrogenphosphat umfasst.

7. Verfahren nach einem der vorstehenden Ansprüche, **dadurch gekennzeichnet, dass** das Medium einen pH-Wert zwischen 3 und 10, bevorzugt zwischen 5 und 7 aufweist.

8. Verfahren nach einem der vorstehenden Ansprüche, **dadurch gekennzeichnet, dass** die Kultivierung zwischen 10°C und 40°C erfolgt, bevorzugt zwischen 25°C und 35°C.

9. Verfahren nach einem der vorstehenden Ansprüche, **dadurch gekennzeichnet, dass** die Kultivierung für 1 bis 10 Tage erfolgt, bevorzugt für 3 bis 9 Tage.

10. Verfahren nach einem der vorstehenden Ansprüche, **dadurch gekennzeichnet, dass** der Mikroorganismus zur Gattung *Schizochytrium, Thraustochytrium* oder *Ulkenia* gehört.

11. Verfahren nach einem der vorstehenden Ansprüche, **dadurch gekennzeichnet, dass** der Mikroorganismus *Ulkenia* spec. SAM 2179 ist.

12. Verfahren nach einem der vorstehenden Ansprüche, **dadurch gekennzeichnet, dass** der Mikroorganismus *Schizochytrium* spec. SR 21 ist.

13. Verwendung eines Kulturmediums umfassend als das wesentliche Mittel zur pH-Wert-Stabilisierung CaCO₃, das mit einem Gehalt von 3 - 15 g/l im Kulturmedium vorliegt, zur Kultivierung von Mikroorganismen der Ordnung Thraustochyriales.

## Claims

1. Process for the cultivation of microorganisms of the order Thraustochytriales, **characterized in that** the microorganisms are cultured in a fermentation medium which includes, as the essential means for stabilizing the pH, CaCO₃ at the content of 3-15 g/l in the fermentation medium, where CaCO₃ is an essential means for stabilizing the pH when the amount of the difference of the pH values which can be measured with or without the addition of acids - in each case with the inventive addition of CaCO₃ - equals 1 or less.

2. Process according to Claim 1, where the microorganisms generate a production of more than 25, preferably more than 35 and very especially preferably more than 45% by weight of oil per unit weight dry biomass.

3. Process according to Claim 1, where the microorganisms generate a production of more than 10, preferably more than 14, especially preferably more than 18 and very especially preferably more than 22% by weight of docosahexaenoic acid (DHA) per unit weight dry biomass.

4. Process according to one of the preceding claims, where the microorganisms generate a production of more than 1%, preferably more than 2%, especially preferably more than 3% and very especially preferably more than 4% by weight of docosapentaenoic acid (DPA) per unit weight dry biomass.

5. Process according to Claim 1 or 2, where 3 g/l to 15 g/l, preferably 4 g/l to 12 g/l, especially preferably 5 g/l to 10 g/l and very especially preferably 7.5 ± 0.5 g/l CaCO₃ are added to the medium.

6. Process according to one of Claims 1 to 5, **characterized in that** the medium comprises glucose, corn steep liquor, magnesium chloride, calcium chloride, calcium carbonate, sodium sulphate, ammonium sulphate and potassium hydrogen phosphate.

7. Process according to one of the preceding claims, **characterized in that** the medium has a pH of between 3 and 10, preferably between 5 and 7.

8. Process according to one of the preceding claims, **characterized in that** culturing is performed at between 10°C and 40°C, preferably between 25°C and 35°C.

9. Process according to one of the preceding claims, **characterized in that** culturing is performed for 1 to 10 days, preferably for 3 to 9 days.

10. Process according to one of the preceding claims, **characterized in that** the microorganism belongs to the genus *Schizochytrium, Thraustochytrium* or *Ulkenia.*

11. Process according to one of the preceding claims, **characterized in that** the microorganism is *Ulkenia* spec. SAM 2179.

12. Process according to one of the preceding claims, **characterized in that** the microorganism is *Schizochytrium* spec. SR 21.

13. Use of a culture medium comprising, as the essential means for stabilizing the pH, CaCO₃, present in the culture medium at a content of 3-15 g/l, for culturing microorganisms of the order Thraustochyriales.

## Revendications

1. Procédé pour cultiver des micro-organismes de l'ordre des thraustochytriales, **caractérisé en ce que** l'on cultive les micro-organismes dans un milieu de fermentation, qui présente comme agent essentiel pour la stabilisation de la valeur de pH du CaCO₃, qui est présent dans le milieu de fermentation avec une teneur de 3 - 15 g/l, dans lequel le CaCO₃ est un agent essentiel pour la stabilisation de la valeur de pH lorsque le montant de la différence des valeurs de pH, qui peuvent être mesurées avec ou sans addition d'acides - chaque fois avec l'addition de CaCO₃ selon l'invention - est inférieure ou égale à 1.

2. Procédé selon la revendication 1, dans lequel les micro-organismes réalisent une production de plus de 25, de préférence de plus de 35 et de préférence encore de plus de 45 % en poids d'huile par unité pondérale de biomasse sèche.

3. Procédé selon la revendication 1, dans lequel les micro-organismes réalisent une production de plus de 10, de préférence de plus de 14, de préférence encore de plus de 18, et encore de préférence de plus de 22 % en poids d'acide docosahexaénoïque (DHA) par unité pondérale de biomasse sèche.

4. Procédé selon l'une quelconque des revendications précédentes, dans lequel les micro-organismes réalisent une production de plus de 1 %, de préférence de plus de 2 %, de préférence encore de plus de 3 %, et encore de préférence de plus de 4 % d'acide docosapentaénoïque (DPA) par biomasse sèche.

5. Procédé selon la revendication 1 ou 2, dans lequel on ajoute au milieu 3 g/l à 15 g/l, de préférence 4 g/l à 12 g/l, de préférence encore 5 g/l à 10 g/l, et encore de préférence 7,5 ± 0,5 g/l de CaCO₃.

6. Procédé selon l'une quelconque des revendications 1 à 5, **caractérisé en ce que** le milieu contient du glucose, de la liqueur de maïs, du chlorure de magnésium, du chlorure de calcium, du carbonate de calcium, du sulfate de sodium, du sulfate d'ammonium et du potassium hydrogénophosphate.

7. Procédé selon l'une quelconque des revendications précédentes, **caractérisé en ce que** le milieu présente une valeur de pH comprise entre 3 et 10, de préférence entre 5 et 7.

8. Procédé selon l'une quelconque des revendications précédentes, **caractérisé en ce que** l'on effectue la culture entre 10°C et 40°C, de préférence entre 25°C et 35°C.

9. Procédé selon l'une quelconque des revendications précédentes, **caractérisé en ce que** l'on effectue la culture pendant 1 à 10 jours, de préférence pendant 3 à 9 jours.

10. Procédé selon l'une quelconque des revendications précédentes, **caractérisé en ce que** le micro-organisme appartient au genre *Schizochytrium, Thraustochytrium* ou *Ulkenia.*

11. Procédé selon l'une quelconque des revendications précédentes, **caractérisé en ce que** le micro-organisme est le *Ulkenia* spec. SAM 2179.

12. Procédé selon l'une quelconque des revendications précédentes, **caractérisé en ce que** le micro-organisme est le *Schizochytrium* spec. SR 21.

13. Utilisation d'un milieu de culture comprenant comme agent essentiel pour la stabilisation de la valeur de pH du CaCO₃, qui est présent avec une teneur de 3 - 15 g/l dans le milieu de culture, pour la culture de micro-organismes de l'ordre des Thraustochytriales.
